# EUROPÄISCHE PATENTSCHRIFT

(11) **EP 3 433 597 B1**
(45) Veröffentlichungstag und Bekanntmachung des Hinweises auf die Patenterteilung: **26.06.2019**
(21) Anmeldenummer: 17712473.2
(22) Anmeldetag: 21.03.2017
(51) Int. Cl.: G01N 1/28, G01N 33/38, G01N 15/02, G01N 25/20, G01N 25/48

(54) **VERFAHREN UND VORRICHTUNG ZUR BESTIMMUNG DES SPEZIFISCHEN MAHLWIDERSTANDS DURCH KALORIMETRISCHE MESSUNG**
METHOD AND DEVICE FOR DETERMINING THE SPECIFIC GRINDING RESISTANCE BY A CALORIMETRIC MEASUREMENT
APPAREIL ET PROCÉDÉ POUR LA DÉTERMINATION DE RESISTANCE AU BROYAGE AU MOYEN DE MESURE CALORIMÉTRIQUE

(30) Priorität: 22.03.2016 DE 102016105319
(43) Veröffentlichungstag der Anmeldung: 30.01.2019
(73) Patentinhaber: thyssenkrupp Industrial Solutions AG, 45143 Essen (DE); thyssenkrupp AG, 45143 Essen (DE)
(72) Erfinder: ENDERS, Michael, 48143 Münster (DE)
(74) Vertreter: thyssenkrupp Intellectual Property GmbH
(86) Internationale Anmeldenummer: PCT/EP2017/056646
(87) Internationale Veröffentlichungsnummer: WO 2017/162639

(56) Entgegenhaltungen:
- LIU C ET AL: "Effects of the granularity of raw materials on the hydration and hardening process of calcium phosphate cement", BIOMATERI, ELSEVIER SCIENCE PUBLISHERS BV., BARKING, GB, Bd. 24, Nr. 23, 31. Oktober 2003 (2003-10-31), Seiten 4103-4113, XP004436344, ISSN: 0142-9612, DOI: 10.1016/S0142-9612(03)00238-2
- CAMIRE C L ET AL: "Correlating crystallinity and reactivity in an alpha-tricalcium phosphate", BIOMATERIALS, ELSEVIER SCIENCE PUBLISHERS BV., BARKING, GB, Bd. 26, Nr. 16, 30. Juni 2005 (2005-06-30) , Seiten 2787-2794, XP027768012, ISSN: 0142-9612 [gefunden am 2005-06-01]
- NOCUN-WCZELIK W ET AL: "Calorimetry and other methods in the studies of expansive cement hydrating mixtures", JOURNAL OF THERMAL ANALYSIS AND CALORIMETRY, KLUWER ACADEMIC PUBLISHERS, DORDRECHT, NL, Bd. 109, Nr. 2, 3. April 2012 (2012-04-03) , Seiten 529-535, XP035090692, ISSN: 1572-8943, DOI: 10.1007/S10973-012-2379-2
- NOCUN-WCZELIK W ET AL: "Calorimetry and other methods in the studies of expansive cement hydrating mixtures", JOURNAL OF THERMAL ANALYSIS AND CALORIMETRY, KLUWER ACADEMIC PUBLISHERS, DORDRECHT, NL, vol. 109, no. 2, 3 April 2012 (2012-04-03) , pages 529-535, XP035090692, ISSN: 1572-8943, DOI: 10.1007/S10973-012-2379-2
- LIU C ET AL: "Effects of the granularity of raw materials on the hydration and hardening process of calcium phosphate cement", BIOMATERI, ELSEVIER SCIENCE PUBLISHERS BV., BARKING, GB, vol. 24, no. 23, 1 October 2003 (2003-10-01), pages 4103-4113, XP004436344, ISSN: 0142-9612, DOI: 10.1016/S0142-9612(03)00238-2
- CAMIRE C L ET AL: "Correlating crystallinity and reactivity in an alpha-tricalcium phosphate", BIOMATERIALS, ELSEVIER SCIENCE PUBLISHERS BV., BARKING, GB, vol. 26, no. 16, 1 June 2005 (2005-06-01), pages 2787-2794, XP027768012, ISSN: 0142-9612 [retrieved on 2005-06-01]

## Beschreibung

Die Erfindung betrifft ein Verfahren und eine Vorrichtung zur Bestimmung des spezifischen Mahlwiderstands von zu zerkleinerndem, mineralischem Mahlgut für die Herstellung von Bindemitteln.

Mahlanlagen bzw. Mühlen zur Zerkleinerung von mineralischem Mahlgut erfordern neben einer sehr robusten Konstruktion und entsprechend verschleißgeschützten Zerkleinerungswerkzeugen einen relativ großen Energieaufwand für die Zerkleinerungs- bzw. Mahlarbeit. So stellt insbesondere das Mahlen von Zementklinker eine besonders energieaufwendige und damit teure Herstellungsstufe bei der Herstellung von Zement dar, zumal hier besonders große Partikelfeinheiten gefordert sind. Moderne Zemente werden heute mit dem Rückstandswert auf dem 45-µm-Sieb charakterisiert. Typische Rückstandswerte liegen bei 0 - 25 % Rückstand auf dem 45µm-Sieb. Man ist daher bestrebt, die Mahlbarkeit der verschiedenen Klinkerarten bereits bei der Rohmaterialzusammenstellung sowie während des Brenn- und/oder Kühlprozesses - soweit möglich und sinnvoll - im Sinne einer leichteren Mahlbarkeit zu beeinflussen. Aus diesem Grunde hat es sich als zweckmäßig erwiesen, den Zementklinkers im laufenden Prozess zu beproben und dessen Mahlwiderstand und damit den zu erwartenden spezifischen Kraftbedarf etwa einer Zementmühle vorauszubestimmen. Mit dem spezifischen Mahlwiderstand kann der Einfluss der chemischen Zusammensetzung, der Rohmaterialien und der Prozessbedingungen, wie Kühlung oder Brennstoff, noch während der Klinkerherstellung in gewisser Weise (üblicherweise mit dem Ziel eines geringeren Energieverbrauchs) gesteuert werden.

Die Vorausbestimmung des Mahlwiderstandes kann aber auch beispielsweise in solchen Anlagen von Nutzen sein, in denen Kompositzemente beispielsweise durch Zumahlung fremdbezogener Stoffe, wie Hochofenschlacke, Puzzolan, Kalkstein und dgl. zu Zement vermahlen werden. In diesem Falle besteht somit die Möglichkeit, bei der Zusammenstellung der verschiedenen Mahlgutkomponenten u. a. auch den Mahlwiderstand des Endprodukts und damit auch die Zerkleinerungsenergie zu reduzieren. In entsprechender Weise kann die Vorausbestimmung des Mahlwiderstandes auch für die Zerkleinerung von anderen mineralischen Mahlgütern, z. B. Gesteine, Erze oder dgl. von Bedeutung sein.

Diese Vorausbestimmung der Mahlbarkeit bzw. des Mahlwiderstandes erfolgt heutzutage in der Praxis fast ausschließlich mit mechanischen Untersuchungsverfahren. Hierbei wird beispielsweise eine Klinkerprobe in einer Labormühle gemahlen und die für verschiedene Mahlfeinheiten notwendige Mahlenergie gemessen. Zu diesen aus der Praxis bekannten Verfahren zählen der sog. Zeiseltest und der Labor-Kugelmühlentest. Beide Verfahren bestimmen die Energieaufnahme durch den Klinker in einem simulierten Mahlprozess.

Die Zunahme der spezifischen Klinkeroberfläche (in Blaine nach EN 196-6) wird in einer Regression in Bezug zu dem Energieverbrauch gesetzt. Der Blainewert hat hier den Vorteil die gesamte Kornverteilung in einem einzigen Zahlenwert zum Ausdruck zu bringen. Die für unterschiedliche Klinker erforderliche spezifische Mahlarbeit kann dann für eine Normfeinheit - in der Regel 3000 cm²/g - interpoliert und als Maß der spezifischen Klinkermahlbarkeit bestimmt werden. Analog kann das Verfahren auch für andere Materialien eingesetzt werden.

Die Mahlbarkeit ist ein wichtiges Maß zur Auslegung von Industriemühlen. Im Falle der Portlandzemente, die nur aus Klinker und Sulfatträger bestehen, wurde verbreitet allein der Blainewert als Maß zur Bewertung der Mahlbarkeit benutzt. Der Blainewert an der Komponente Klinker ist relativ leicht und sicher mit einem Fehler in der Größenordnung von 3 % zu bestimmen. Zusammen mit der Energieaufnahme ergibt sich jedoch häufig ein deutlich erhöhter Fehler von ca. 5% für die spezifische Mahlbarkeit.

Die in der Vergangenheit für die Zementmahlung häufig eingesetzte Kugelmühle weist aufgrund der attritierenden Zerkleinerung im Vergleich zu den auf Druckzerkleinerung basierenden Vertikalrollenmühlen und Gutbettwalzenmühlen einen höheren Feinanteil auf. Der höhere Feinanteil wirkt positiv auf den zur Korngrößenbeschreibung verwandten Blainewert. Moderne mit Hochleistungssichtern ausgestattete Mahlaggregate (Vertikalrollenmühlen, Gutbettwalzenmühlen) weisen häufig geringere Feinanteile und insgesamt steilere Kornverteilungen auf. Der geringere Feinanteil und die steilere Kornverteilung wirken sich aber ungünstig auf den Blainewert aus. Daher werden meist auf modernen Mühlen höhere Feinheiten zur Erzeugung von Zementen gleicher Leistung - zum Beispiel hinsichtlich der Festigkeitsentwicklung - vorgegeben.

Global werden heute überwiegend Kompositzemente aus Klinker, Sulfatträger und weiteren Zumahlstoffen (Flugaschen, Hüttensand, Kalkstein, Puzzolan) erzeugt. Der spezifische Energiebedarf für die Vermahlung der Kompositzemente wird über ein gewichtetes Mittel der Mahlbarkeit der Einzelkomponenten abgeschätzt. Hierzu sind aber Versuche mit mehreren Zementbestandteilen notwendig, was zu einem hohen Aufwand führt. Die gemeinsame Vermahlung der Komponenten unterschiedlicher spezifischer Mahlbarkeit in Testverfahren, als auch in der Zementmühle ist fehlerbehaftet, da der materialabhängige spezifische Mahlwiderstand zu unterschiedlichen Kornverteilungskurven der Einzelkomponenten in den Kompositzementen führt. Zum Beispiel wird in einem Kalksteinzement die Kalksteinkomponente meist schneller zerkleinert als die für die Festigkeitsentwicklung wichtige Klinkerkomponente. Daher ist der an der Gesamtprobe des Kompositzements ermittelte Blainewert nicht mehr charakteristisch für die Festigkeitsentwicklung des Zements und stellt daher keinen zuverlässigen Wert für die Bestimmung der Mahlbarkeit dar.

In der DE 197 45 793 A1 wurde daher ein Verfahren zur Vorausbestimmung des Mahlwiderstandes von Mahlgut angegeben, wobei zunächst eine Kalibrationskurve von wenigstens einem repräsentativen Vergleichsmahlgut von einer Anzahl von physikalisch aufbereiteten und ausgewerteten Proben erstellt wird, um anschließend die wesentlichen Eigenschaften der Hauptmineralien des neuen Mahlgutes diffraktometrisch und durch automatische Profilanalyse labormäßig zu ermitteln.

Druckschrift D1, LIU C ET AL: "Effects of the granularity of raw materials on the hydration and hardening process of calcium phosphate cement",BIOMATERI, ELSEVIER SCIENCE PUBLISHERS BV., 2003-10-31, Seiten 4103-4113, zeigt ein Verfahren zur Bestimmung der spezifischen Partikelgröße von zu zerkleinerndem, mineralischem Mahlgut für die Herstellung von Bindemitteln. Der Gegenstand der Erfindung unterscheidet sich dadurch, dass es ein Verfahren zur Bestimmung des Mahlwiderstands ist statt zur Bestimmung der Partikelgröße wie in Druckschrift D1.

Der Erfindung liegt nun die Aufgabe zugrunde, ein neues Verfahren zur Bestimmung des spezifischen Mahlwiderstands von zu zerkleinerndem, mineralischem Mahlgut für die Herstellung von Bindemitteln anzugeben, dass sich für Klinker allein und für Bindemittel zusammengesetzt aus Komponenten mit unterschiedlicher spezifischer Mahlbarkeit besonders eignet.

Erfindungsgemäß wird diese Aufgabe durch die Merkmale der Ansprüche 1 und 11 gelöst.

Beim erfindungsgemäßen Verfahren wird eine Probe des mineralischen Mahlgutes in mehrere Mahlgut-Teilproben aufgeteilt, die in einer Labormühle mit unterschiedlicher Intensität gemahlen werden und wobei weiterhin für jede der mit unterschiedlicher Mahlintensität gemahlenen Mahlgut-Teilproben der Wärmefluss durch eine kalorimetrische Messung als Maß für den spezifischen Mahlwiderstand ermittelt wird.

Das erfindungsgemäße Verfahren stellt somit nicht mehr auf die Zunahme der spezifischen Oberfläche des Mahlgutes in Abhängigkeit der Mahlintensität, sondern auf die Zunahme an Reaktivität des Mahlgutes ab.

Bindemittel, wie beispielsweise Zement, reagieren beim Abbinden mit Wasser exotherm. Die dabei freiwerdende Hydratationswärme korreliert mit den zu erzielenden Festigkeitswerten des Zements. Mit Hilfe der Kalorimetrie kann die Hydratationswärme ermittelt werden, die ein Maß für die hydraulische Reaktivität darstellt, welche die Leistungsfähigkeit eines Zementes charakterisiert. Die hydraulische Reaktivität eines Zementes ist eine integrale Eigenschaft, die relativ das Reaktionsverhalten verschiedener Zemente beschreibt. So wird beispielsweise bei sonst gleicher Zusammensetzung der Zement mit einer rascheren Festigkeitsentwicklung als der Zement mit einer höheren Reaktivität charakterisiert. Eine Möglichkeit der direkten Erfassung der Reaktivität von Zementen ist die isothermale Wärmeflusskalorimetrie, dabei wird die bei der Hydratation eines Bindemittels freigesetzte Wärme über die Zeit ermittelt. Aus der Abbildung des Wärmeflusses über die Zeit sind Zeiten mit maximalem Wärmefluss ebenso abzulesen, wie die kumulative Wärmeabgabe zu einem festgelegten Zeitpunkt.

Die Erfindung betrifft ferner eine Vorrichtung zur Durchführung des obigen Verfahrens zur Bestimmung des spezifischen Mahlwiderstands mit
- wenigstens einer Dosiereinrichtung zur Aufteilung einer Probe des mineralischen Mahlguts in mehrere Mahlgut-Teilproben,
- wenigstens einer auf unterschiedliche Mahlintensität einstellbaren Labormühle sowie
- wenigstens einem Wärmeflusskalorimeter zur Ermittlung des Wärmeflusses der mit unterschiedlicher Mahlintensität gemahlenen Mahlgut-Teilproben.

Vorteilhafte Ausgestaltungen der Erfindung sind Gegenstand der weiteren Ansprüche.

Die Vorrichtung kann optional wenigstens einer Dosiereinrichtung zur Zugabe von Sulfatverbindungen als Erstarrungsregler umfassen.

Bei der kalorimetrischen Messung wird vorzugsweise der Zeitpunkt des maximalen Wärmeflusses oder der kumulierte Wärmefluss zu einem Referenzzeitpunkt ermittelt.

Um die verschiedenen Mahlgut-Teilproben besser miteinander vergleichen zu können, wird die Probe zunächst durch Fraktionierung eines Ausgangsmaterial so aufbereitet, dass sich in der Probe eine definierte Korngrößenverteilung zwischen einer minimalen und einer maximalen Korngröße, beispielsweise zwischen 0.8mm und 1mm, durch Fraktionierung einstellt. Die fraktionierte Probe wird dann in mehrere Mahlgut-Teilproben aufgeteilt und in einer Labormühle mit unterschiedlicher Mahlintensität gemahlen. Eine Labormühle kann beispielsweise durch eine Scheibenschwingmühle oder eine Kugelmühle gebildet werden. Sie zeichnet sich gegenüber einer Industriemühle vor allem dadurch aus, dass sie relativ kleine Mengen von beispielsweise wenigen Gramm bis einigen Kilos, beispielsweise 4g bis 5kg pro Stunde, verarbeitet.

Die unterschiedlichen Mahlintensitäten für die Mahlung der Mahlgut-Teilproben kann vorzugsweise durch Variation der Umdrehungsgeschwindigkeit der Labormühle oder durch Variation der Aufbereitungszeit in der Labormühle eingestellt werden.

Zur Ermittlung des Wärmeflusses der mit unterschiedlicher Mahlintensität gemahlenen Mahlgut-Teilproben kommt vorzugsweise ein Wärmeflusskalorimeter zum Einsatz, wobei die bei der Hydratation einer Mahlgut-Teilprobe freigesetzte Wärme über die Zeit ermittelt wird. Dabei ist eine Mahlgut-Teilprobe mit einer höheren Reaktivität durch eine schnellere und größere kumulierte Wärmeabgabe bis zu einem festgelegten Referenzzeitpunkt charakterisiert. Als Ergebnis lassen sich der Wärmefluss der mit unterschiedlichster Mahlintensität gemahlenen Mahlgut-Teilproben gegenüber der jeweils aufgewandten Mahlintensität auftragen, wobei die sich dabei ergebende Kurve abhängig von der gewählten Bezugsgröße entweder die Änderung des Zeitpunkts des maximalen Wärmeflusses bei der Reaktion angibt oder aber die kumulierte Wärmeabgabe an einem Referenzzeitpunkt gegenüber der Mahlintensität widerspiegelt.

Bei dem mineralischen Mahlgut kann es sich insbesondere um einen mit einem Sulfatträger als Abbinderegler versetzten Klinker oder Hüttensand allein oder gegebenenfalls mit einem Anreger (z.B. Sulfat, Ca(OH)₂) versetzt oder einen Zement, insbesondere Kompositzement, handeln.

Die vorliegende Erfindung bezieht sich weiterhin auf ein Verfahren zur industriellen Herstellung eines Bindemittels aus zu zerkleinerndem, mineralischem Mahlgut, wobei auf ermittelte Informationen aus dem oben beschriebenen Verfahren zur Bestimmung des spezifischen Mahlwiderstands zurückgegriffen wird.

So können die beim oben beschriebenen Verfahren zur Bestimmung des Mahlwiderstands ermittelten Informationen bei der industriellen Herstellung eines Bindemittels, beispielsweise von Zement, wie folgt eingesetzt werden:
1. Durch die Auswahl der Rohmaterialien für die Klinkerproduktion kann die Reaktivität des Klinkers beeinflusst werden. Man kann diesen Reaktivitätsunterschied für eine gegebene Mahlintensität abbilden.
2. Durch die Auswahl der Rohmaterialien für die Klinkerproduktion kann die Mahlbarkeit des Klinkers beeinflusst werden. Man kann die Mahlintensität für eine gewünschte Reaktivität abbilden.
3. Durch die Auswahl der Brennstoffe kann die Mahlbarkeit beeinflusst werden. Man kann die Mahlintensität für eine gewünschte Reaktivität abbilden.
4. Mit der Abbildung von Reaktivität und Mahlintensität können in einem Prozessschritt die spezifischen Kosten einen Zements hinsichtlich der Zusammensetzung des Zements aus unterschiedlichen mineralischen Mahlgütern und der benötigten Mahlenergie optimiert werden.
5. Mit der Abbildung von Reaktivität und Mahlintensität kann in einem Prozessschritt die Wärmeabgabe eines Zements hinsichtlich der Zusammensetzung des Zements aus unterschiedlichen mineralischen Mahlgütern und die benötigte Mahlenergie optimiert werden. Dies ist zum Beispiel für die Produktion von Zementen mit niedriger Wärmeabgabe (LH-Zemente) hilfreich.
6. Mit der Bestimmung des Wärmeflusses kann in einem Prozessschritt die Leistungsfähigkeit eines Zements (beschrieben als freigesetzte Wärme) und die dafür benötigte Mahlenergie bei der Herstellung von Zementen bestehend aus unterschiedlichen mineralischen Mahlgütern ermittelt und optimiert werden. Zum Beispiel können, abhängig von tagesaktuellen Elektrizitätspreisen, die Zusammensetzung und/oder die Feinheit eines Kompositzements in Richtung niedriger Energiekosten oder in Richtung niedriger spezifischer Materialkosten gegeneinander optimiert werden.

Weitere Vorteile der Erfindung werden anhand der nachfolgenden Beschreibung und der Zeichnung näher erläutert.

In der Zeichnung zeigen
- Fig. 1: ein schematisches Blockschaltbild einer Vorrichtung zur erfindungsgemäßen Bestimmung des spezifischen Mahlwiderstands,
- Fig. 2: ein Diagramm für verschiedene Proben, wobei die Änderung des Wärmeflusses gegenüber der Mahlintensität aufgetragen ist und
- Fig. 3.: ein reales Beispiel, in dem die Wirkung einer unterschiedlichen Aufbereitungszeit auf die nach 72 Stunden erreichte kumulierte Wärmeabgabe an zwei Beispielen dargestellt ist.

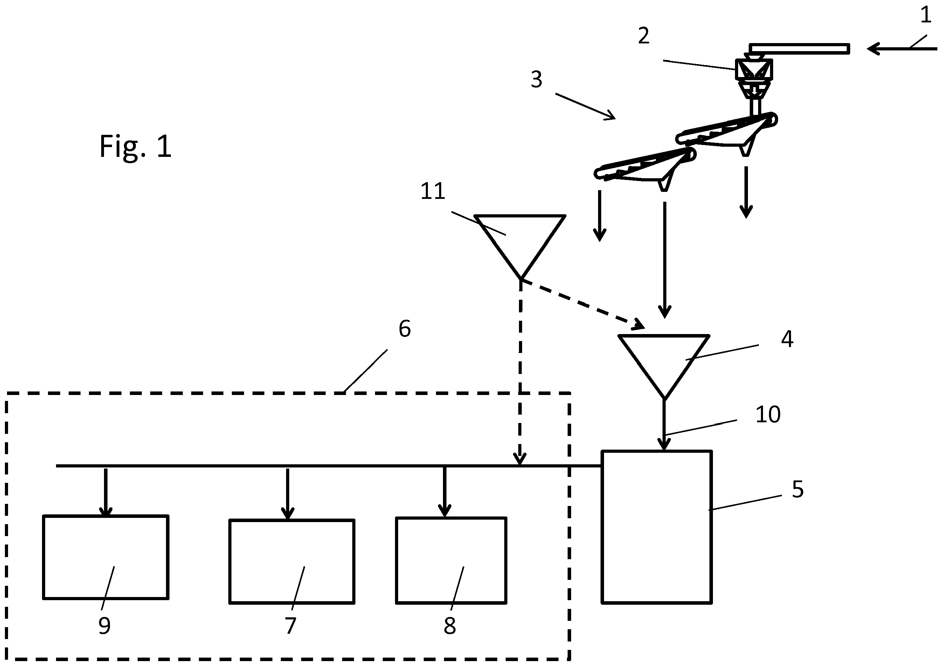

Die in Fig. 1 dargestellte Vorrichtung zur erfindungsgemäßen Bestimmung des spezifischen Mahlwiderstands besteht im Wesentlichen aus einer einen Brecher 2 und eine Siebmaschine 3 aufweisenden Fraktioniereinrichtung, einem Vorratsbehälter 4 und einer Labormühle 5 sowie eine Analyseeinrichtung 6. Die Analyseeinrichtung 6 umfasst insbesondere ein Wärmeflusskalorimeter 7 und kann ferner eine Einrichtung 8 zur Bestimmung der Korngröße und einer Einrichtung 9 der spezifischen Oberfläche aufweisen.

Bei der Durchführung des Verfahrens zur Bestimmung des Mahlwiderstands wird eine Probe 1 eines mineralischen Mahlguts zunächst im Brecher 2 und der Siebmaschine 3 durch Fraktionierung so aufbereitet, dass sich in der fraktionierten Probe eine definierte Korngrößenverteilung einstellt. Diese fraktionierte Probe wird im Zwischenbehälter 4 gelagert. Das Brechen und/oder das Sieben kann dabei manuell oder vollautomatisch durchgeführt werden. Zweckmäßigerweise sollte die fraktionierte Probe eine Korngrößenverteilung von 0.8 bis 1mm aufweisen, da diese Korngrößenverteilung auch bei anderen Untersuchungsmethoden (mikroskopische Untersuchung von Klinker, Zeisel-Test) üblich ist. Es sind aber natürlich auch andere eindeutig definierbare Kornfraktionen denkbar.

Im nachfolgenden Arbeitsschritt erfolgt die Mahlung in der Labormühle 5, die über den Vorratsbehälter 4 mit Mahlgut-Teilproben 10 beaufschlagt wird. Der Vorratsbehälter 4 ist so ausgelegt, dass er eine ausreichende Anzahl an Mahl-Teilproben zulässt. Die Aufgabemenge der Mahlgut-Teilproben 10 in die Labormühle 5 wird bevorzugt gravimetrisch und weniger bevorzugt volumetrisch bestimmt. Die Aufgabemenge kann beispielsweise 12g betragen, wobei eine Genauigkeit von mindestens 0.05g bevorzugt wird. Bei einer volumetrischen Dosierung wird über die Schüttdichte das Volumen des Dosiergefäßes festgelegt.

Zur Einstellung eines geeigneten Reaktionsverhaltens kann es notwendig sein, aus einem Behälter 11 einen Sulfatträger, beispielweise Gips oder Halbhydrat oder Anhydrit oder in Ausnahmefällen Alkalisulfate, volumetrisch oder bevorzugt gravimetrisch zuzudosieren. Typischerweise werden zwischen 0 und 10 % Sulfatträger zudosiert. In Einzelfällen, beispielsweise bei Sulfathüttenzement oder Sulfoaluminatzement oder einem sulfatischen Bindemittel können abweichend auch höhere Zudosierungen an Sulfatträger eingestellt werden.

Die anschließende Mahlung in der Labormühle 5 erfolgt mit einer voreingestellten Mahlintensität, also insbesondere mit einer vorgegebenen Mahldauer und einer vorgegebenen Umdrehungszahl der Mühle. Die gemahlene Mahlgut-Teilprobe wird abdosiert und direkt der Analyseeinrichtung 6 zugeführt, wo insbesondere eine manuelle oder automatisierte Messung des Wärmeflusses (Hydratationswärme) mittels des Wärmeflusskalorimeters 7 erfolgt. Optional können zusätzlich die Korngröße und die spezifische Oberfläche bestimmt werden.

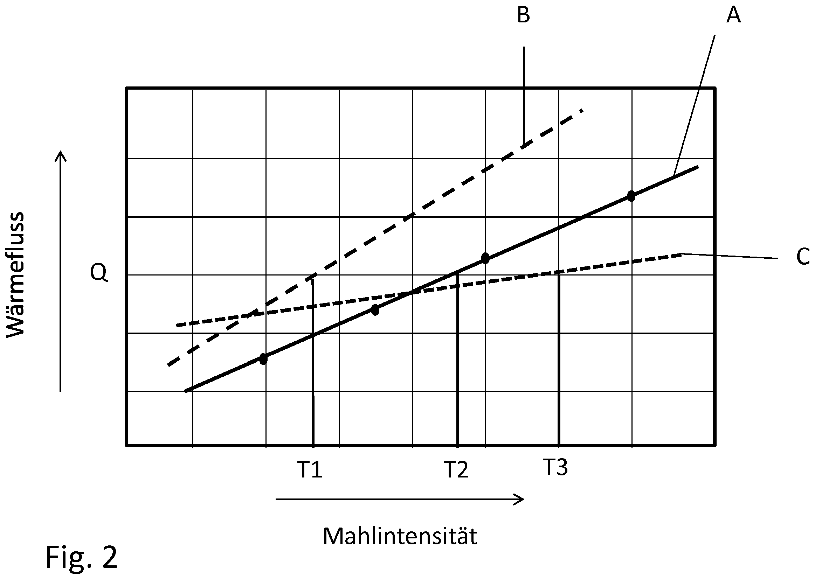

Anschließend wird die Labormühle 5 wiederum aus dem Vorratsbehälter 4 mit einer weiteren Mahlgut-Teilprobe 10 beschickt, wobei eine Mahlung mit abweichender Mahlintensität gewählt wird. Gegenüber der vorhergehenden Mahlung kann die Änderung der Mahlintensität beispielsweise durch Veränderung der Mahldauer und/oder der Umdrehungszahl der Labormühle 5 erfolgen. Das Produkt der erneuten Mahlung wird wiederum in der Analyseeinrichtung 6 insbesondere hinsichtlich des Wärmeflusses im Wärmeflusskalorimeter 7 untersucht. Nach einer Mindestzahl an untersuchten Mahlgut-Teilproben, die mit unterschiedlicher Mahlintensität gemahlen wurden, kann in einer Regression die Mahlintensität gegenüber dem Wärmefluss aufgetragen werden. So ergibt sich bei der Mahlung von vier Mahlgut-Teilproben 10 einer bestimmten Probe 1 beispielsweise die in Fig. 2 dargestellte Kurve A. Bei der Untersuchung von Proben anderer mineralischer Mahlgüter ergeben sich beispielsweise die Kurven B und C.

Jede der Kurven A, B, C bildet somit unterschiedliche Mahlgüter ab, von denen jeweils Proben gezogen wurden. Jede Kurve wurde anhand von Messungen einer bestimmten Anzahl an Mahlgut-Teilproben der jeweils zugehörigen Probe ermittelt.

Betrachtet man zunächst nur die Kurve A in Fig. 2 ist zunächst festzustellen, dass der Wärmefluss bzw. die kumulative Wärmeabgabe an einem Referenzzeitpunkt mit steigender Mahlintensität bei den untersuchten Mahlgut-Teilproben ansteigt. Dies ist auf die vergrößerte Oberfläche der gemahlenen Mahlgut-Teilproben bei zunehmender Mahlintensität zurückzuführen. Die Steigung der Kurve A beschreibt dabei den Zusammenhang zwischen der in die Probe eingebrachten Mahlintensität und dem dabei durch die vergrößerte Oberfläche erzielten Reaktivitätsgewinn. Für den Vergleich mit anderen Mahlgütern ist es zweckmäßig eine Vergleichsgröße Q für den Wärmefluss festzulegen. Diese Vergleichsgröße kann entweder ein Zeitpunkt mit einem maximalen Wärmefluss oder der kumulierte Wärmefluss an einem Referenzzeitpunkt sein. Im vorliegenden Beispiel der Kurve A kann dann durch eine mathematische Regression oder eine grafische Lösung (Fig. 2) die Mahlintensität T2 ermittelt werden, die zum Erreichen des Wärmeflusses Q aufgewandt werden muss. Neben der Vergleichsgröße Q ist auch die Anwendung weiterer Vergleichsgrößen oder die Steigung der Kurve als Bewertungskriterium denkbar.

Bei der Kurve B handelt es sich um eine Kurve, die sich bei einer Probe eines anderen Mahlguts ergeben hat. Im Vergleich zur Kurve A weist sie einen höheren Startwert und eine größere Steigung auf. Dies bedeutet zunächst, dass die der Kurve B zugrundeliegende Probe bei geringerer Mahlintensität bereits eine höhere Reaktivität aufweist. Der Startpunkt ist also ein Maß für die Reaktivität des Ausgangsmaterials. Der steilere Verlauf der Kurve B belegt zudem, dass für einen gegebenen Reaktivitätsanstieg eine geringere Mahlintensität benötigt wird. Zusammenfassend ist die der Kurve B zugrundeliegende Probe reaktiver und es ist eine Reaktivitätssteigung bei der Vermahlung durch einen geringeren Mahlenergieeinsatz möglich. Das Material ist somit leichter auf Reaktivität mahlbar und hat dementsprechend einen geringeren spezifischen Mahlwiderstand. Dies ist auch an der Vergleichsgröße Q feststellbar, die belegt, dass diese gewählte Reaktivität bei der Kurve B bereits bei einer Mahlintensität T1 erreicht wird, während bei der Kurve A zugrundeliegenden Probe eine deutlich höhere Mahlintensität T2 erforderlich ist.

Betrachtet man nur die Kurve C, ist festzustellen, dass diese gegenüber den beiden Kurven A und B einen noch höheren Startwert aufweist. Das Material ist also reaktiver als das den Kurven A und B zugrundeliegende Mahlgut. Die Steigung der Kurve C ist jedoch kleiner, was bedeutet, dass der Reaktivitätsgewinn aus einer gegebenen zusätzlichen Mahlintensität kleiner ist als bei den beiden anderen Materialien. Der Wärmefluss bei der Vergleichsgröße Q wird auch erst bei einer Mahlintensität T3 erreicht. Die der Kurve C zugrundeliegende Probe weist also den höchsten spezifischen Mahlwiderstand auf, sodass die Aktivierung der Reaktivität einen hohen Energiebetrag erfordert. Man spricht also davon, dass das der Kurve C zugrundeliegende Material schwer mahlbar ist.

Vergleicht man das bekannte Verfahren nach Zeisel mit dem oben vorgeschlagenen erfindungsgemäßen Verfahren ergeben sich folgende Unterschiede:
Bei dem bekannten Verfahren nach Zeisel bestimmt allein die durch Aufmahlung entstehende Oberfläche, die üblicherweise nach Blaine bestimmt wird, den spezifischen Mahlwiderstand als Energiebedarf für eine Vergleichsfeinheit in kwh/t. Der Zeiseltest ergibt durch die Bestimmung einer elektrischen Leistungsaufnahme direkt die spezifische Mahlenergie. Dabei wird üblicherweise ein Oberflächenwert von 3000 Blaine zum Vergleich unterschiedlicher Proben herangezogen. Die Oberfläche nach Blaine ist jedoch nicht immer eine zuverlässige Indikation für die Reaktivitätszunahme bei einer Zerkleinerung einer Probe. In der Anwendung eines Zements ist jedoch die Reaktivität das entscheidende Maß, da unterschiedliche Bindemittel bei gleicher Oberfläche durchaus unterschiedliche Reaktivitäten erwarten lassen. Das erfindungsgemäße Verfahren ermöglicht den Reaktivitätsgewinn (Hydratationswärme) in Bezug auf den Energieverbrauch (Mahlintensität) darzustellen. Man kann also für eine bestimmte Zielreaktivität den ermittelten Energieverbrauch ermitteln. Das oben beschriebene erfindungsgemäße Verfahren ermöglicht die Optimierung der Mahlbarkeit, insbesondere der Klinkermahlbarkeit im Zementwerk, in dem durch regelmäßige Bestimmung der spezifischen Mahlbarkeit und einem Abgleich mit der Mahlgutzusammensetzung eine empirische Optimierung der chemischen Zusammensetzung des Mahlgutes und eine optimierte Auswahl der ggf. erforderlichen Zusatzstoffe vorgenommen wird. Im Vergleich mit Industrieproben kann zudem eine Kalibration des Systems in Richtung einer spezifischen Mahlenergie vorgenommen werden.

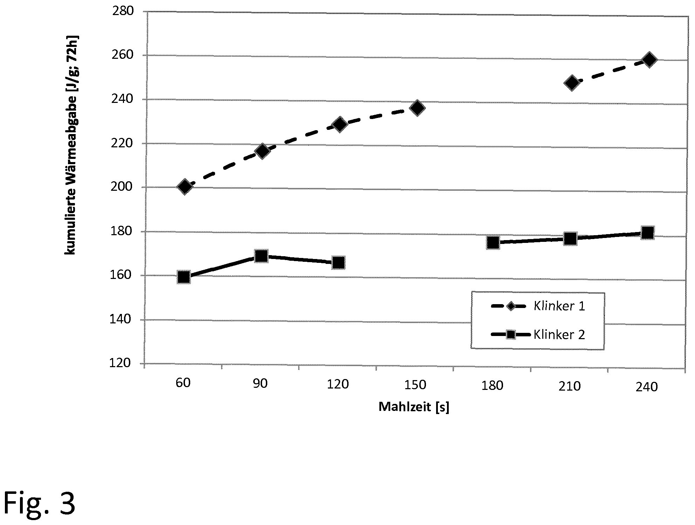

Fig. 3 zeigt ein Beispiel für das Verfahren an zwei verschiedenen Klinkern (Klinker 1 und Klinker 2). Das Diagramm lässt erkennen, dass der Klinker 1 bei gleicher Mahlzeit deutlich reaktiver als der Klinker 2 ist. Selbst eine bis zu viermal längere Mahlzeit führ nicht zu einer wesentlich besseren Reaktivität. Selbst bei einer vierfach längeren Mahlzeit liegt die Reaktivität des Klinkers 2 immer noch unter dem Startwert des Klinkers 1. Die Reaktivität des Klinkers 1 lässt sich hingegen durch einen längere Mahlzeit nochmals deutlich steigern.

Ein wichtiger Faktor bei der Bestimmung der spezifischen Herstellungskosten von Bindemitteln, insbesondere Zement, ist neben den spezifischen Materialkosten, vor allem die Energie für die Mahlung. So könnte man beispielsweise eine günstige, schwer mahlbare Komponente gegen eine teure, leichter mahlbare Komponente austauschen. So ist es insbesondere auch möglich, mit der spezifischen Mahlbarkeit für eine vergleichsbare Leistungsfähigkeit des Bindemittels eine Entscheidung darüber zu treffen, welches Material in einem neuen Kompositzement mit dem Ziel einer kostengünstigen Herstellung verwendet werden soll.

## Patentansprüche

1. Verfahren zur Bestimmung des spezifischen Mahlwiderstands von zu zerkleinerndem, mineralischem Mahlgut für die Herstellung von Bindemitteln, wobei eine Probe (1) des mineralischen Mahlguts in mehrere Mahlgut-Teilproben (10) aufgeteilt wird, die in einer Labormühle (5) mit unterschiedlicher Mahlintensität gemahlen werden,
**dadurch gekennzeichnet, dass**
weiterhin für jede der mit unterschiedlicher Mahlintensität gemahlenen Mahlgut-Teilproben (10) der Wärmefluss durch eine kalorimetrische Messung als Maß für den spezifischen Mahlwiderstand ermittelt wird.

2. Verfahren nach Anspruch 1, **dadurch gekennzeichnet, dass** bei der kalorimetrischen Messung der Zeitpunkt des maximalen Wärmeflusses ermittelt wird.

3. Verfahren nach Anspruch 1, **dadurch gekennzeichnet, dass** bei der kalorimetrischen Messung der kumulierte Wärmefluss zu einem Referenzzeitpunkt ermittelt wird.

4. Verfahren nach Anspruch 1, **dadurch gekennzeichnet, dass** die Probe (1) durch Fraktionierung eines Ausgangsmaterials so aufbereitet wird, dass sich in der Probe eine definierte Korngrößenverteilung zwischen einer minimalen und einer maximalen Korngröße durch Fraktionierung eingestellt.

5. Verfahren nach Anspruch 1, **dadurch gekennzeichnet, dass** die unterschiedlichen Mahlintensitäten durch Variation der Umdrehungsgeschwindigkeit der Labormühle (5) eingestellt werden.

6. Verfahren nach Anspruch 1, **dadurch gekennzeichnet, dass** die unterschiedlichen Mahlintensitäten durch Variation der Aufbereitungszeit in der Labormühle (5) eingestellt werden.

7. Verfahren nach Anspruch 1, **dadurch gekennzeichnet, dass** zur Ermittlung des Wärmeflusses der mit unterschiedlicher Mahlintensität gemahlenen Mahlgut-Teilproben (10) ein Wärmeflusskalorimeter (7) zum Einsatz kommt.

8. Verfahren nach Anspruch 1, **dadurch gekennzeichnet, dass** der Wärmefluss der mit unterschiedlicher Mahlintensität gemahlenen Mahlgut-Teilproben (10) gegenüber der jeweils aufgewandten Mahlintensität aufgetragen werden, wobei die sich dabei ergebene Kurve die Änderung des Wärmeflusses gegenüber der Mahlintensität widerspiegelt.

9. Verfahren nach Anspruch 1, **dadurch gekennzeichnet, dass** es sich bei dem mineralischen Mahlgut um einen mit einem Sulfatträger als Abbinderegler versetzten Klinker oder Hüttensand oder Zement handelt.

10. Verfahren zur industriellen Herstellung eines Bindemittels aus zu zerkleinerndem, mineralischem Mahlgut, wobei auf ermittelte Information aus dem Verfahren zur Bestimmung des spezifischen Mahlwiderstands gemäß Anspruch 1 zurückgegriffen wird.

11. Vorrichtung zur Durchführung des Verfahrens zur Bestimmung des spezifischen Mahlwiderstands nach Anspruch 1 mit
- wenigstens einer Dosiereinrichtung zur Aufteilung einer Probe (1) des mineralischen Mahlguts in mehrere Mahlgut-Teilproben (10),
- wenigstens einer auf unterschiedliche Mahlintensität einstellbaren Labormühle (5) sowie
- wenigstens einem Wärmeflusskalorimeter (7) zur Ermittlung des Wärmeflusses der mit unterschiedlicher Mahlintensität gemahlenen Mahlgut-Teilproben (10).

12. Vorrichtung nach Anspruch 11, **dadurch gekennzeichnet, dass** der Dosiereinrichtung eine Fraktionierungseinrichtung zur Fraktionierung einer Probe auf eine definierte Korngrößenverteilung zwischen einer minimalen und einer maximalen Korngröße vorgeschaltet ist.

## Claims

1. Method for determining the specific milling resistance of mineral milling stock to be comminuted for the production of binders, where a sample (1) of the mineral milling stock is divided into a plurality of milling stock subsamples (10) which are milled with different milling intensities in a laboratory mill (5),
**characterized in that**
the heat flow as measure of the specific milling resistance is additionally determined by means of calorimetric measurement for each of the milling stock subsamples (10) milled with a different milling intensity.

2. Method according to Claim 1, **characterized in that** the point in time of maximum heat flow is determined in the calorimetric measurement.

3. Method according to Claim 1, **characterized in that** the cumulated heat flow up to a reference point in time is determined in the calorimetric measurement.

4. Method according to Claim 1, **characterized in that** the sample (1) is treated by fractionation of a starting material so that a defined particle size distribution between a minimum particle size and a maximum particle size is established in the sample by fractionation.

5. Method according to Claim 1, **characterized in that** the different milling intensities are set by varying the speed of rotation of the laboratory mill (5).

6. Method according to Claim 1, **characterized in that** the different milling intensities are set by varying the treatment time in the laboratory mill (5).

7. Method according to Claim 1, **characterized in that** a heat flow calorimeter (7) is used for determining the heat flow of the milling stock subsamples (10) milled with different milling intensities.

8. Method according to Claim 1, **characterized in that** the heat flow of the milling stock subsamples (10) milled with different milling intensities is plotted against the milling intensity applied in each case, where the resulting curve reflects the change in the heat flow as a function of the milling intensity.

9. Method according to Claim 1, **characterized in that** the mineral milling stock is a clinker admixed with a sulphate carrier as setting regulator or slag sand or cement.

10. Process for the industrial production of a binder from mineral milling stock to be comminuted, wherein information determined from the method for determining the specific milling resistance according to Claim 1 is employed.

11. Apparatus for carrying out the method for determining the specific milling resistance according to Claim 1, comprising
- at least one metering device for dividing a sample (1) of the mineral milling stock into a plurality of milling stock subsamples (10),
- at least one laboratory mill (5) which can be set to different milling intensities and
- at least one heat flow calorimeter (7) for determining the heat flow of the milling stock subsamples (10) milled with different milling intensities.

12. Apparatus according to Claim 11, **characterized in that** the metering device is installed downstream of a fractionating device for fractionating a sample to a defined particle size distribution between a minimum particle size and a maximum particle size.

## Revendications

1. Procédé de détermination de la résistance au broyage spécifique d'un matériau minéral à concasser destiné à la fabrication de liants, dans lequel un échantillon (1) du matériau minéral est divisé en une pluralité de sous-échantillons de matériau (10), lesquels sont broyés avec des intensités de broyage différentes dans un broyeur de laboratoire (5),
**caractérisé en ce qu'**en outre, pour chacun des sous-échantillons de matériau (10) broyés avec différentes intensités de broyage, le flux thermique est déterminé par mesure calorimétrique en tant que mesure de la résistance au broyage spécifique.

2. Procédé selon la revendication 1, **caractérisé en ce que** l'instant où le flux thermique est maximal est déterminé lors de la mesure calorimétrique.

3. Procédé selon la revendication 1, **caractérisé en ce que** le flux thermique cumulé est déterminé à un instant de référence lors de la mesure calorimétrique.

4. Procédé selon la revendication 1, **caractérisé en ce que** l'échantillon (1) est préparé par fractionnement d'un matériau de départ de manière à ce qu'une distribution granulométrique définie soit réglée par fractionnement dans l'échantillon entre des granulométries minimale et maximale.

5. Procédé selon la revendication 1, **caractérisé en ce que** les différentes intensités de broyage sont réglées en faisant varier la vitesse de rotation du broyeur de laboratoire (5).

6. Procédé selon la revendication 1, **caractérisé en ce que** les différentes intensités de broyage sont réglées en faisant varier le temps de préparation dans le broyeur de laboratoire (5).

7. Procédé selon la revendication 1, **caractérisé en ce qu'**un calorimètre à flux thermique (7) est utilisé pour déterminer le flux thermique des sous-échantillons de matériau (10) broyés avec différentes intensité de broyage.

8. Procédé selon la revendication 1, **caractérisé en ce que** le flux thermique des sous-échantillons de matériau (10) broyés avec différentes intensités de broyage est appliqué en fonction de l'intensité de broyage respectivement appliquée, dans lequel la courbe ainsi obtenue reflète la variation du flux thermique en fonction de l'intensité de broyage.

9. Procédé selon la revendication 1, **caractérisé en ce que** le matériau minéral est un clinker ou un laitier de haut fourneau ou un ciment mélangé à un support de sulfate en tant que régulateur de prise.

10. Procédé de fabrication industrielle d'un liant à partir d'un matériau minéral à concasser, dans lequel on a recours à des informations obtenues à partir du procédé de détermination de la résistance au broyage spécifique selon la revendication 1.

11. Dispositif pour la mise en oeuvre du procédé de détermination de la résistance au broyage spécifique selon la revendication 1, comprenant
- au moins un dispositif de dosage destiné à diviser un échantillon (1) du matériau minéral en plusieurs sous-échantillons (10),
- au moins un broyeur de laboratoire (5) réglable à différentes intensités de broyage, et
- au moins un calorimètre à flux thermique (7) destiné à déterminer le flux thermique des sous-échantillons de matériau (10) broyés avec des intensités de broyage différentes.

12. Dispositif selon la revendication 11, **caractérisé en ce qu'**un dispositif de fractionnement destiné à fractionner un échantillon selon une distribution granulométrique définie entre des granulométries minimale et maximale est monté en amont du dispositif de dosage.
